# EUROPEAN PATENT SPECIFICATION

(11) **EP 2 456 330 B1**
(45) Date of publication and mention of the grant of the patent: **24.07.2013**
(21) Application number: 10728026.5
(22) Date of filing: 03.06.2010
(51) Int. Cl.: A61L 15/42, B32B 5/26, B32B 27/02, B32B 27/32, B32B 27/36, A41D 31/00, A61L 15/26, A61B 19/08, A41D 13/12

(54) **BLOOD RESISTANT AND VIRAL RESISTANT COMPOSITE FABRICS**
BLUT UND VIREN UNDURCHLASSIGE VERBUNDSTOFFE
TISSUS COMPOSITES IMPERMEABLES AU SANG ET AUX VIRUS

(30) Priority: 03.04.2010 US 320709 P
(43) Date of publication of application: 30.05.2012
(73) Proprietor: Tredegar Film Products Corporation, Richmond, VA 23225 (US)
(72) Inventor: RAY, Carl, Douglas, Chesterfield VA 23832 (US)
(74) Representative: Brunetti, Fabrizio
(86) International application number: PCT/US2010/037186
(87) International publication number: WO 2010/141670

(56) References cited:
- EP-A2- 1 695 819
- WO-A1-2004/058500
- WO-A2-2005/034659
- WO-A2-2006/076722
- US-A1- 2004 219 337

## Description

### Cross-Reference to Related Applications

This application claims the benefit of the filing date of US Provisional Application no. 61/ 320709 filed April 3, 2010, which claims the benefit of the filing date of US Provisional Application no. 61/ 217587 filed June 2, 2009.

### Background of the Disclosure

The disclosure concerns composite fabrics, and more particularly, to composite fabrics having blood and viral barrier properties that make the fabric suitable for use as a protective garment in healthcare applications.

In the healthcare field, there is an awareness of the need to provide protection to healthcare workers against the spread of communicable viral or blood-borne diseases, such as AIDS and hepatitis. Protective fabrics for use in surgical gowns, masks, drapes and other protective apparel have been developed for this purpose. Regulations and standards such as the OHSA Universal Precautions act and the current proposed Surgical Gown classification standards under development by the Association for the Advancement of Medical Instrumentation (AAMI) further contribute to the awareness of this need.

Industry standards for assessing the barrier properties of protective fabrics against penetration by blood and viral agents include ASTM F1670, Standard Test Method for Resistance of Materials Used in Protective Clothing to Penetration by Synthetic Blood, and ASTM F 1671, Standard Test Method for Resistance of Materials Used in Protective Clothing to Penetration by Blood-Borne Pathogens Using Phi-x 174 Bacteriophage Penetration as a Test System.

According to the AMMI Technical Information Report TIR11:2005, a surgical gown, or other protective apparel product, is given a level 4 classification, the highest and best rating, if it has an accepted quality level (AQL) of no more than 4% with respect to both the ASTM F1670 and ASTM 1671 test procedures. The AQL is defined as the quality level that for the purpose of sampling inspection is the limit of a satisfactory process average. See ANSI/AAMI PB70:2003. The Food and Drug Administration (FDA) requires an acceptable quality level (AQL) for making a claim to passing ASTM F1671 of 30 out of 34 samples tested (88.23% pass rate). In practice a much smaller frequency of rejections would be desirable.

Protective fabrics are available that meet the above barrier standards (ASTM F 1670 and ASTM F1671) at a reasonable cost, but these fabrics are typically plastic coated fabrics that offer little or no breathability. Their lack of breathability significantly contributes to the discomfort and heat stress of the wearer. One way that gown manufacturers try to improve comfort is by using the coated fabrics only in the frontal and arm areas of the gown. However, this practice compromises protection in other areas of the body.

A common method used by industry to determine the breathability of a barrier fabric is Moisture Vapor Transfer Rate (MVTR) as determined by ASTM E96, Standard Test Methods for Water Vapor Transmission of Materials. There are breathable barrier fabrics available that provide moisture vapor transfer while passing ASTM F1670 and ASTM F1671. These barrier fabrics are based on perfluoroethylene or copolyester films and membranes. However, because of their expense, they are typically used in protective garments that are reusable, and have limited applicability as disposable garments.

One way of obtaining favorable economics in a breathable composite material utilizes a process wherein a polymer containing a mechanical pore forming agent is extruded in a single pass onto a nonwoven fabric and subsequently incrementally stretched in the cross machine and/or machine direction. The resulting composite material is microporous. It is impervious to the passage of liquids while the presence of micropores provides moisture vapor or air permeability. For example, micropores in the range of about 0.1 µm to about 1 µm can be formed in the composite. Such technologies are described in U.S. 5,865,926; U.S. 6,258,308; and US 2003/0124324.

A disadvantage of this type of coating process as compared to a lamination process such as that described in U.S. 5,409,761 is that the extrusion coating process has a tendency to form pinholes or discontinuities in the fabric. Such pinholes can cause failure of both ASTM F1670 and ASTM F1671. If the pinholes are sufficiently small, e.g. microscopic, the coating may pass the ASTM F1670 blood penetration test, but would nonetheless fail the more stringent viral penetration test of ASTM F1671.

US Patent Publication 2004/0219337 and US Patent Publication 2003/0124324 address this concern by applying a microporous film layer on either side of the nonwoven web. As mentioned in these publications, the basic concept is that by utilizing low basis weight nonwoven webs and two microporous coatings, the likelihood of having pinhole formation is reduced and the possibility alignment through the composite of pinholes that may form is remote. However, having a film layer positioned adjacent to the skin of the user reduces the comfort level of garments made from the fabrics. In addition, the dual coatings add to the cost of making the product and also tend to reduce the drape, hand and flexibility of the material.

WO 2006/076722 discloses a laminate containing a film layer that is positioned between an absorbent non woven layer and a repellent nonwoven layer. The film layer is a water impermeable, vapor permeable monolithic material that is estrusion coated onto the absorbant nonwoven layer.The repellent nonwoven layer is then adhesivelt laminated to the opposite side of the monolithic film layer. The resulting laminate has a vapor transmission rate of at least 3000g/m²/24 hr and passes the blood transmission and viral transmission tests of ASTm F1670 and F1671 respectively.

There is a need in the art for fabric that will meet the stringent requirements of the ASTM F1671 viral penetration test while maintaining high breathability and exceptional comfort.

### Summary of the Disclosure

The disclosure provides a composite fabric that meets the blood and viral barrier tests described above, and has excellent breathability.

In one embodiment, the composite fabric meets the level 4 blood barrier test.

In some embodiments, the composite fabric has a MVTR in excess of 1500 gm/m²/24hrs, or in excess of 3000 gm/m²/24hrs, or in excess of 4500 gm/m²/24hrs or in excess of 6500 gm/m²/24hrs.

In one embodiment, the composite fabric comprises a nonwoven web of fusable fibers having a breathable monolithic coating on at least one surface thereof.

In one embodiment, the monolithic coating comprises a polyesterether or a highly substituted ionomer.

In one embodiment, the nonwoven web is fully calendered and is free of fibers protruding through the monolithic coating.

These and other features of the embodiments will become apparent upon a further reading of the specification and the appended claims.

### Brief Description of the Drawing Figures

Figure 1 is a side elevation view of one embodiment of a composite fabric in accordance with the disclosure.

Figure 2 is a side elevation view of another embodiment of a composite fabric in accordance with the disclosure.

### Detailed Description

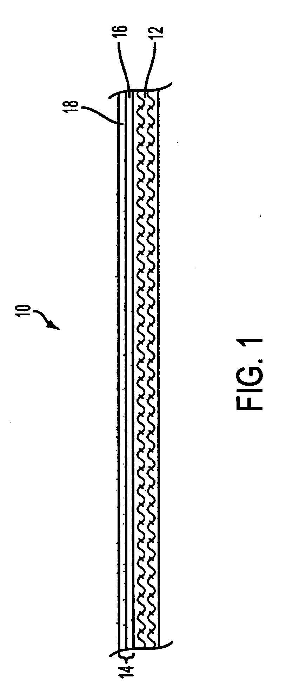

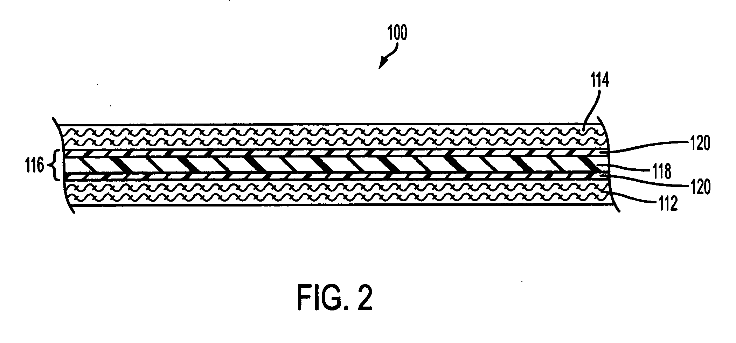

With reference to Figure 1, in one embodiment, the composite fabrics 10 comprise a nonwoven fibrous web 12 and a monolithic, breathable coating 14 on at least one surface of the nonwoven web 12. With reference to Figure 2, a second embodiment of the composite fabric is illustrated. The composite fabric 100 comprises a two nonwoven webs 112, 114 disposed on either side of a monolithic, breathable coating layer 116.

### Fibrous Web

The nonwoven webs in the embodiments may be of any standard construction known in the art. Nonwoven fabrics are comprised of fibers arranged in a random or non-repeating pattern. Nonwoven webs can generally be classified as continuous or staple fiber webs. Continuous fiber webs are webs made in processes where fibers are generated and deposited onto a conveyor or mat in a generally continuous process. Spunbonding and meltblowing are examples of processes that produce continuous fiber webs. The term "spunbonded fibers" refers to small diameter fibers that are formed by extruding a molten thermoplastic material as filaments from a plurality of fine, usually circular, capillaries of a spinneret with the diameter of the extruded filaments then being rapidly reduced as by, for example, eductive drawing or other well-known spunbonding mechanisms. Other shapes of the capillaries used to make the fibers, and thus the shape of the fibers themselves, are also known and useful.

Meltblown and meltblowing refer to fibers formed by extruding a molten thermoplastic material through a plurality of fine, usually circular, die capillaries as molten threads or filaments into a high velocity gas (e.g., air) stream that attenuates the filaments of molten thermoplastic material to reduce their diameter, which may be to a microfiber diameter (i.e., an average diameter not greater than about 100 µm). Thereafter, the meltblown fibers are carried by the high velocity gas stream and are deposited on a collecting surface to form a web of randomly dispersed meltblown fibers. Other shapes of the capillaries used to make the fibers, and thus the shape of the fibers themselves, are also known and useful.

Staple fiber webs are webs made by depositing fibers of discrete lengths onto a mat or conveyor. Examples of webs having staple fibers include carded webs. Carding is a process for making fibrous webs in which the fibers are aligned either parallel or randomly in the direction that the web is produced by the carding machine. The carding machine is a device having a series of drums or rollers with wire projections or metal teeth that separate the fibers from impurities. The fibers in carded webs are predominantly aligned in the machine direction, but may also be randomly oriented.

Once the fibers are made and deposited by any of the above processes, the individual fibers are formed into a coherent web by any one or more of a variety of processes, such as thermal bonding (calendaring), hyrdoentangling, resin bonding, or other methods known in the art.

The fibers can be made from a variety of thermoplastic polymers, including polyolefins such as polyethylene and polypropylene, polyesters, copolyesters, polyvinyl acetate, polyamides, copolyamides, polystyrenes, polyurethanes, elastomeric materials such as styrene block copolymers available from, for example Kraton Polymers LLC, or ultralow density polyethylene resins or olefinic block copolymer resins available from, for example, DOW Chemicals, and copolymers of any of the foregoing such as vinyl chloride/vinyl acetate, and the like. Webs of glass fibers can also be employed in certain embodiments.

Suitable thermoplastic fibers can be made from a single polymer (monocomponent fibers), or can be made from more than one polymer (e.g., bicomponent fibers). For example, "bicomponent fibers" can refer to thermoplastic fibers that comprise a core fiber made from one polymer that is encased within a thermoplastic sheath made from a different polymer. The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer.

Bicomponent fibers can include sheath/core fibers having the following polymer combinations: polyethylene/polypropylene, polyethylvinyl acetate/polypropylene, polyethylene/polyester, polypropylene/polyester, copolyester/ polyester, styrene block copolymer elastomer/ polypropylene, polyester/ low density polyethylene and the like. The bicomponent fibers can be concentric or eccentric, referring to whether the sheath has a thickness that is even, or uneven, through the cross-sectional area of the bicomponent fiber. Eccentric bicomponent fibers can be desirable in providing more compressive strength at lower fiber thicknesses.

In the case of thermoplastic staple fibers for carded nonwoven fabrics, their length can vary depending upon the particular melt point and other properties desired for these fibers. Typically, these thermoplastic fibers have a length from about 0.3 to about 7.5 cm long, preferably from about 0.4 to about 3.0 cm long. The properties, including melt point, of these thermoplastic fibers can also be adjusted by varying the diameter (caliper) of the fibers. The diameter of these thermoplastic fibers is typically defined in terms of either denier (grams per 9000 meters) or decitex (grams per 10,000 meters). Depending on the specific arrangement within the structure, suitable thermoplastic fibers can have a decitex in the range from well below 1 decitex, such as 0.4 decitex, up to about 20 decitex.

The term "polymer" includes homopolymers, copolymers, such as, for example, block, graft, random and alternating copolymers, terpolymers, etc., and blends and modifications thereof. Furthermore, unless otherwise specifically limited, the term "polymer" is meant to include all possible geometrical configurations of the material, such as isotactic, syndiotactic and atactic or random symmetries.

The nonwoven webs may also be subjected to standard finishing techniques. In a preferred embodiment, the nonwoven web is a fully calendered web.

The monolithic coating 14, 116 on the web should be as thin as possible for several reasons. First, thicker coatings tend to have lower breathability versus thinner coatings. Secondly, thicker coatings tend to produce fabrics that are stiffer and have less desirable drape than thinner coatings. And, of course, thinner coatings require less material and thus are less costly to produce. In some embodiments, the monolithic coating is between 10 and 100 µm thick, and all ranges therebetween. In some embodiments, the monolithic coating is between 25 and 75 µm thick. In other embodiments, the coating may only be about 10 µm thick.

To maintain the blood and viral resistance, it is important that the surface of then nonwoven web that is to receive the coating be free of any fibers that might protrude through the monolithic coating. Fibers that protrude beyond the thickness of the monolithic coating create pinholes that allow for the passage of viral compounds and also openings for blood to pass. Selecting a nonwoven web made of continuous fibers can be an advantage because they tend to have fewer or no free fibers ends protruding from the surface of the web. However, even continuous fiber nonwoven webs may have fiber loops that extend above the surface of the web, creating the possibility for pinhole creation.

Thus, it is an important aspect of the composites that the nonwoven web are selected and/or processed to ensure that no fiber ends or fiber loops protrude above the surface of the web beyond the thickness of the monolithic coating. For this reason, it is preferred that the nonwoven web be fully calendered under heat and pressure sufficient to eliminate any such surface fibers. In addition, it is preferred that the nonwoven web comprise fusible fibers. The term "fusible fibers" refers to nonwoven webs that contain fibers made of materials having different melting temperatures. During the calendaring process, the fibers with the lower melting temperature will melt or soften and fuse to the other fibers in the web, thus increasing the internal bonding within the web and ensuring that the web surface remains smooth and free of any fibers that might protrude above the monolithic coating.

Examples of fusible fiber webs include bi-component fiber webs in which a higher melting polymer, such as polyester or polypropylene, is encased in a sheath of a lower melting polymer, such as polyethylene. Other examples include nonwoven webs containing both polypropylene fibers and polyethylene fibers, or webs made of fibers that are half one polymer and half another, so long as the polymer, or polymer blend, used for each half had a different melting temperature. Bicomponent fiber webs provide the best results and are therefore preferred. Especially preferred for medical applications are fibrous webs made of bi-component fibers of polyester with a sheath of low density polyethylene because such webs can be sterilized using gamma radiation.

### Monolithic Coating

The composites fabrics have a monolithic coating 14,116 on at least one surface of the nonwoven web. Many prior art composites with blood and/or viral resistance utilize microporous films or coatings. Such films comprise a particulate material, such as calcium carbonate, distributed in a polymer matrix, After the film is formed, it is stretched to pull the polymer away from the particulate material, thus creating a porous network that is too small for liquids to pass, but large enough for the transmission of water vapor. Monolithic films and coatings, on the other hand, have pores with cross-sectional sizes on a molecular scale formed by a polymerization process. The pores serve as conduits by which water (or other liquid) molecules can disseminate through the film. Vapor transmission occurs through a monolithic film or coating as a result of a concentration gradient across the monolithic film. This process is referred to as activated diffusion. As water (or other liquid) evaporates on one side of the film, the concentration of water vapor increases. The water vapor condenses and solubilizes on the surface of the film. As a liquid, the water molecules dissolve into the film. The water molecules then diffuse through the monolithic film and re-evaporate into the air on the side having a lower water vapor concentration.

The monolithic coating 14 is preferably applied using a vacuum coating technique, as taught in US 5,753,342; US 5,762,643; US 5,660,882; and US 6,211,102/. In the vacuum coating process, a molten polymer is extruded from a die onto a substrate - in this case a calendered nonwoven web - while the substrate is supported on a perforated forming screen. Vacuum is applied to the opposite side of the substrate, which serves to pull the coating into the substrate, thus embedding the fibers in the coating. The vacuum coating technique is preferred because it results in a composite that has improved toughness and resistance to abrasion. Other suitable, but less preferred, techniques for applying the monolithic coating or film include adhesive lamination, ultrasonic binding, vacuum lamination, and extrusion coating.

Suitable resins for use as the monolithic coatings include Pebax® polyether block amide elastomers from Arkema, Inc.; Hytrel ® polyester elastomers from DuPont; and Entire® Breathe, also from DuPont, which is a highly substituted polyolefin ionomer. The Entira® Breathe ionomer is particularly preferred because of its lower cost and better compatibility with most nonwoven webs.

To enhance the adhesion between the nonwoven fabric and the monolithic coating, it may be desirable to apply a thin layer of adhesion promoters, such as ethylene methyl acrylate, between the monolithic coating and the nonwoven web. This may conveniently be accomplished by spraying the adhesion promoting material onto the fibrous web or applying the monolithic coating 14, 116 as a coextrusion of the adhesion promoting layer 16,120 and the monolithic resin layer 18, 118 as seen in Figures 1 and 2. Care must be taken to keep the adhesion promoting layer as thin as possible to ensure that the composite maintains a high MVTR. Because a layer of adhesion promoter can negatively impact breathability, it is preferable that the adhesion promoter is blended with the monolithic composition in percentages that are consistent with good breathability yet enhances the bond to the nonwoven. Concentrations of 15-25% by weight of adhesion promoter, preferably 18-22%, have proven advantageous.

### Processing

To improve the tactile properties of the composite, it may be advantageous to incrementally stretch the composite. Incremental stretching is a known process in which a sheet material is stretched by passing it through a nip formed by toothed rollers that intermesh with one another. As the sheet material passes through the rollers, frictional forces cause the sheet to be "gripped" where it contacts the apex of the teeth on the rollers. This then becomes a grip point or friction point where the material remains relatively stationary. The sheet material thus assumes a sinusoidal path through the nip, which stretches the sheet in the regions between adjacent grip points. By bringing the rollers closer together, the degree of intermeshing (i.e., the depth of engagement) increases and results in a greater web path, thus increasing the amount of stretch. The grip points remain relatively stationary and thus do not stretch, or stretch very little relative to the adjacent areas. Accordingly, the sheet material is stretched in increments that correspond to the location of the teeth on the rollers where the film was gripped.

The degree of stretch imparted in the process is related to the size and shape of the teeth on the gear rollers, the pitch of the teeth, and the depth of engagement. Typically, however, the process results in alternating areas of very high stretch and either no stretch or minimal stretch. For example, it is possible for regions of the material to be stretched to 100 or 200% and an adjacent area not be stretched at all. Unlike tenter stretchers or machine direction stretching techniques, the overall dimensions of the sheet after incremental stretching can be only 2-6% more than before stretching, yet the sheet material will have the same properties (breathability) as if it had been stretched to several hundred percent in a tenter frame.

The composite may be incrementally stretched in the machine direction, the cross direction, or both in the embodiments. Various apparatus are known in the art to make the incremental stretching process more uniform across the web. For example, US 6,368,444 teach such an apparatus. Such apparatus may be used to advantage in the embodiments.

### Examples

A series of composite fabrics were prepared and tested for MVTR as well as blood resistance. MVTR was measured using the method described in ASTM E46. Blood resistance was determined using the method described in ASTM F1670. Results are reported in Table 1.

### Example 1

A nonwoven web, NSTCPTE-50 from Shalag Shamir, having a basis weight of 50 grams/m² was passed through a nip formed by two heated rollers to fully calendar the web so that no fibers we extending beyond the surface of the web. A coating comprised of a coextrusion of a microporous layer and a monolithic EMA layer (Exxon TC120) were extruded onto the surface of the nonwoven web and the coated web was subjected to vacuum to draw the coating into the web and encapsulate the fibers. The coating thickness was 23 µm in the final composite.

### Example 2

The process of Example 1 was repeated, except that a hydrophobic additive (Ampacet A101722) was blended into the microporous layer of the coextruded coating.

### Example 3

The process of Example 1 was repeated, except that an ultralow density polyethylene resin (Dow EG8200) was used instead of the EMA.

### Example 4

The process of Example 1 was repeated, except that a highly substituted ionomer (DuPont RENTAS500) was used instead of the EMA.

### Example 5

The process of Example 1 was repeated, except that NSTCPTE-30 (Shalag Shamir), a 30 g/m² calendared bicomponent nonwoven was used instead of NSTCPTE-50, and a polypropylene version of microporous layer was used instead of the polyethylene version of Example 1.

### Example 6

The process of Example 1 was repeated, except that the microporous layer was replaced with 100% Entira® Breathe and the coextruded skin layer was Entira® Breathe modified for adhesion to NSTCPTE-30.

### Example 7

The process of Example 1 was repeated, except that the thickness of the coextruded coating comprised 100% Entira® Breathe as one layer and Entira® Breathe with an EMA adhesion promoter and the total coating thickness was reduced to 10 µm.

**Table 1**

| **Example** | **MVTR (gm/m²/day)** | **Blood Resistance (pass/fail)** |
|---|---|---|
| 1 | 100 | Mixed |
| 2 | 60 | Passed |
| 3 | 75 | Passed |
| 4 | 500 | Passed |
| 5 | 1100 | Mixed |
| 6 | 3600 | Passed |
| 7 | 7000 | Passed |

As seen from Table 1, the examples using a monolithic coating (examples 6 & 7) had a significantly higher MVTR and a consistent pass rate for blood resistance as compared to microporous coatings, even if used with conventional monolithic coatings at the same coating thickness. Of particular note is that Example 7 had only a 10 µm thick coating and yet passed the blood resistance test and had remarkably high MVTR.

### Example 8

A nonwoven web, NSTCPTE-30 from Shalag Shamir, having a basis weight of 30 grams/m² was passed through a nip formed by two heated rollers to fully calendar the web so that no fibers we extending beyond the surface of the web. The web was coated with a coextrusion comprising layer 1 and layer 2 and the coated web was subjected to vacuum to draw the coating into the web and encapsulate the fibers. Layer 1 comprised a blend of Entira® Breathe (70%) blended with EMA (TC120; 30%). Layer 2 comprised 100% Entira® Breathe. The coating was applied at a coating rate of 12 g/m².

Ten samples were randomly selected from the web of Example 8 and subjected to testing for viral resistance using the method of ASTM 1671. Results are reported in Table 2.

**Table 2**

| **Sample No:** | **No. of plaques** | **Visual Penentration** | **Test Result** |
|---|---|---|---|
| 8-1 | None | None | Pass |
| 8-2 | None | None | Pass |
| 8-3 | None | None | Pass |
| 8-4 | None | None | Pass |
| 8-5 | None | None | Pass |
| 8-6 | None | None | Pass |
| 8-7 | None | None | Pass |
| 8-8 | None | None | Pass |
| 8-9 | None | None | Pass |
| 8-10 | Too numerous to count | None | Fail |

### Example 9

A nonwoven web, NSTCPTE-30 from Shalag Shamir, having a basis weight of 30 grams/m² was passed through a nip formed by two heated rollers to fully calendar the web so that no fibers we extending beyond the surface of the web. The web was coated with a coextrusion comprising layer 1 and layer 2 and the coated web was subjected to vacuum to draw the coating into the web and encapsulate the fibers. Layer 1 comprised a blend of Entira® Breathe (70%) blended with EMA (TC120; 30%). Layer 2 comprised 100% Entira® Breathe. The coating was applied at a coating rate of 12 g/m².

### Example 10

A nonwoven web (7705 from DuPont) having a basis weight of 26 grams/m² was coated with a three-layer coextruded film while the nonwoven web was subjected to vacuum pressure of 203mm Hg to draw the coating into the web and encapsulate the fibers. The first layer of the film comprised a blend of Entira® Breathe (80%) blended with EMA (20%). The second and third layers each consisted of 100% Entira® Breathe. The coating was applied at a coating rate of 12 grams/m², with layer 1 comprising about 15 wt% of the three-layer coating. An adhesive was then spry atomized onto the surface of the three-layer film at a weight of 4 grams/ m² and a second web of nonwoven (7705 from DuPont) was adhered to the glue layer. The resulting composite was then passed through a nip formed between two intermeshing gears and was incrementally stretched in the transverse direction to an engagement depth of 1 mm to improve the tactile feel of the web.

### Example 11

Example 10 was repeated except that the resulting composite was incrementally stretched to a depth of engagement of 1.65 mm.

### Example 12

Example 10 was repeated, except that the three-layer coating was applied at a rate of 15 grams/m².

### Example 13

Example 11 was repeated except that the three-layer coating was applied at a rate of 15 grams/m².

### Example 14

A nonwoven web (7720 from DuPont) having a basis weight of 38 grams/m² was coated with a two-layer coextruded film coating while the nonwoven web was subjected to vacuum pressure of 356 mm Hg to draw the coating into the web and encapsulate the fibers. The first layer of the film comprised a blend of Entira® Breathe (80%) blended with EMA (20%) and the second layer consisted of 100% Entira® Breathe. The coating was applied at a coating rate of 14 grams/m², with the first layer comprising about 15 wt% of the coating. An adhesive was then spry atomized onto the surface of the film coating at a weight of 4 grams/ m² and a second web of nonwoven (7705 from DuPont) with a basis weight of 37.3 grams/ m² was adhered to the glue layer. The resulting composite was then passed through a nip formed between two intermeshing gears and was incrementally stretched in the transverse direction to an engagement depth of 1.65 mm to improve the tactile feel of the web.

## Claims

1. A composite fabric comprising a fully calendared nonwoven web of fibers and a monolithic coating on at least one surface of said web, said monolithic coating comprises a polyolefin ionomer; wherein said composite fabric has a MVTR of at least 1500 g/m²/day and wherein the composite passes the ASTM F1670 blood barrier test and the ASTM F 1671 viral barrier test.

2. The composite of claim 1, wherein the nonwoven web comprises bicomponent fibers of polyester and polyethylene.

3. The composite of claim 1, wherein the nonwoven web comprises fusible fibers.

4. The composite of claim 1, wherein the monolithic coating comprises a coextrusion of a monolithic resin layer and an adhesion promoting layer.

5. The composite of claim 1 wherein the composite has a MVTR of at least 3000 g/m²/day.

6. The composite of claim 1 wherein the composite has a MVTR of at least 4500 g/m²/day.

7. The composite of claim 1 wherein the composite has a MVTR of at least 6500 g/m²/day.

8. The composite of claim 1, comprising a first nonwoven web, a first adhesion promoting layer adhered to the first nonwoven web, a monolithic layer comprising a polyolefin ionomer adhered to the first adhesion promoting layer; an adhesive layer adhered to the monolithic layer, and a second nonwoven web adhered to the adhesive layer.

9. The composite of claim 8, wherein the composite is incrementally stretched in at least one direction selected from the machine direction and the cross direction.

10. The composite of claim 1, wherein the monolithic coating comprises a vacuum coated layer which encapsulates at least some of the fibers in the nonwoven web.

## Patentansprüche

1. Verbundstoff, umfassend ein vollständig kalandriertes nichtgewebtes Faservlies und eine monolithische Beschichtung auf wenigstens einer Oberfläche des Vlieses, wobei die monolithische Beschichtung ein Polyolefinionomer umfaßt, wobei der Verbundstoff eine MVTR von wenigstens 1500 g/m²/Tag aufweist und wobei der Verbundstoff den ASTM-F1670-Blutschrankentest und den ASTM-F1671-Virenschrankentest besteht.

2. Verbundstoff nach Anspruch 1, wobei das nichtgewebte Vlies Zweikomponentenfasern aus Polyester und Polyethylen umfaßt.

3. Verbundstoff nach Anspruch 1, wobei das nichtgewebte Vlies Schmelzfasern umfaßt.

4. Verbundstoff nach Anspruch 1, wobei die monolithische Beschichtung eine Coextrusion einer monolithischen Harzschicht und einer Anheftungsvermittlungsschicht umfaßt.

5. Verbundstoff nach Anspruch 1, wobei der Verbundstoff eine MVTR von wenigstens 3000 g/m²/Tag aufweist.

6. Verbundstoff nach Anspruch 1, wobei der Verbundstoff eine MVTR von wenigstens 4500 g/m²/Tag aufweist.

7. Verbundstoff nach Anspruch 1, wobei der Verbundstoff eine MVTR von wenigstens 6500 g/m²/Tag aufweist.

8. Verbundstoff nach Anspruch 1, umfassend ein erstes nichtgewebtes Vlies, eine an dem ersten nichtgewebten Vlies angeheftete erste Anheftungsvermittlungsschicht, eine monolithische Schicht, die ein an der ersten Anheftungsvermittlungsschicht angeheftetes Polyolefinionomer umfaßt, eine an der monolithischen Schicht angeheftete Adhäsionsschicht und ein an der Adhäsionsschicht angeheftetes zweites nichtgewebtes Vlies.

9. Verbundstoff nach Anspruch 8, wobei der Verbundstoff in wenigstens einer Richtung, die aus der Maschinenrichtung oder der Querrichtung ausgewählt ist, schrittweise gestreckt ist.

10. Verbundstoff nach Anspruch 1, wobei die monolithische Beschichtung eine vakuumbeschichtete Schicht umfaßt, die wenigstens einige der Fasern in dem nichtgewebten Vlies verkapselt.

## Revendications

1. Tissu composite comprenant une bande non tissée complètement calandrée de fibres et un revêtement monolithique sur au moins une surface de ladite bande, ledit revêtement monolithique comprend un ionomère de polyoléfine ; dans lequel ledit tissu composite présente un MVTR d'au moins 1 500 g/m²/jour et dans lequel le composite passe le test de barrière au sang ASTM F 1670 et le test de barrière viral ASTM F 1671.

2. Composite selon la revendication 1, dans lequel la bande non tissée comprend des fibres biconstituantes de polyester et de polyéthylène.

3. Composite selon la revendication 1, dans lequel la bande non tissée comprend des fibres fusibles.

4. Composite selon la revendication 1, dans lequel le revêtement monolithique comprend une coextrusion d'une couche de résine monolithique et d'une couche de promotion d'adhérence.

5. Composite selon la revendication 1, dans lequel le composite présente un MVTR d'au moins 3 000 g/m²/jour.

6. Composite selon la revendication 1, dans lequel le composite présente un MVTR d'au moins 4 500 g/m²/jour.

7. Composite selon la revendication 1, dans lequel le composite présente un MVTR d'au moins 6 500 g/m²/jour.

8. Composite selon la revendication 1, comprenant une première bande non tissée, une première couche de promotion d'adhérence collée à la première bande non tissée, une couche monolithique comprenant un ionomère de polyoléfine collée à la première couche de promotion d'adhérence ; une couche adhésive collée à la couche monolithique, et une seconde bande non tissée collée à la couche adhésive.

9. Composite selon la revendication 8, dans lequel le composite est progressivement étiré dans au moins une direction choisie parmi la direction de la machine et la direction transversale.

10. Composite selon la revendication 1, dans lequel le revêtement monolithique comprend une couche déposée sous vide qui encapsule au moins certaines des fibres dans la bande non tissée.
